# EUROPÄISCHE PATENTANMELDUNG

(11) **EP 1 602 327 A1**
(43) Veröffentlichungstag der Anmeldung: **07.12.2005**
(21) Anmeldenummer: 04011527.1
(22) Anmeldetag: 14.05.2004
(51) Int. Cl.: A61B 5/15

(54) **Blutentnahmevorrichtung mit einem Kanülenhalter**

(71) Anmelder: Sarstedt AG & Co., 51588 Nümbrecht (DE)
(72) Erfinder: Sarstedt, Walter, 51588 Nümbrecht (DE)
(74) Vertreter: Valentin, Ekkehard

(57) **Zusammenfassung**

Bei einer Blutentnahmevorrichtung mit einem Kanülenhalter (1), insbesondere für eine Doppelkanüle (3), der ein schwenkbares, bis auf eine zwischen einander gegenüberliegenden Seitenwänden (7a, 7b) vorgesehene Kanüleneintrittsöffnung umfangsgeschlossenes Schutzgehäuse (2) für das freie Kanülenende (4 a) aufweist, sind die freien Enden (8a, 8b) der Seitenwände (7a, 7b) des Schutzgehäuses (2) komplementär zu einem sich weitestgehend über die gesamte Gehäuselänge erstreckenden Kanülen-Einführtrichter (8) ausgeformt.

## Beschreibung

Die Erfindung betrifft eine Blutentnahmevorrichtung mit einem Kanülenhalter, insbesondere für eine Doppelkanüle, der ein schwenkbares, bis auf eine zwischen einander gegenüberliegenden Seitenwänden vorgesehene Kanüleneintrittsöffnung umfangsgeschlossenes Schutzgehäuse für das freie Kanülenende aufweist.

Aus der DE 691 27 906 T2 und DE 692 25 609 T2 sind solche Blutentnahmevorrichtungen mit einem Sicherheits- bzw. Schutzgehäuse bekannt. Damit der Benutzer oder ein Dritter nicht mit der Kanüle bzw. daran verbliebenen Bluttropfen in Berührung kommt, wird das Schutzgehäuse nach Beendigung der Blutentnahme fluchtend zur Kanüle verschwenkt, die danach von dem Schutzgehäuse aufgenommen wird. Das Schutzgehäuse weist als Kanüleneintrittsöffnung einen langgestreckten, offenen Schlitz auf, welcher durch im wesentlichen parallel längs des Gehäuses verlaufenden, beabstandeten Stirnkanten von einander zugewandt abgewinkelten Wänden begrenzt wird. Durch diesen Schlitz tritt die Kanüle ein, wenn das längliche Schutzgehäuse in die mit der Kanüle fluchtende Position geschwenkt wird.

Im Schutzgehäuse selbst sind Zurückhaltemittel vorgesehen, um zu verhindern, dass die eingeschwenkte Kanüle aus dem Schutzgehäuse ungewollt, selbsttätig wieder austritt. Diese Zurückhaltemittel sind in Form von Haken ausgebildet, die in dem von den Schutzgehäusewänden eingeschlossenen Kanal und somit im Inneren des Schutzgehäuses an dessen Bodenwand angeordnet sind. Die Haken weisen jeweils einen an der gleichen Gehäusewand angeordneten Bereich auf, der gegen die Kanüle drückt, wenn das Schutzgehäuse verschwenkt wird, der jedoch in seine ursprüngliche Stellung zurückkehrt, nachdem die Kanüle ihn passiert hat, um hierdurch die Kanüle innerhalb des Gehäuses zurückzuhalten. Bei den bekannten Blutentnahmevorrichtungen ist das Schutzgehäuse über eine Lasche und einen Klemmring entweder stationär oder drehbar an dem Kanülenhalter befestigt.

Die bekannten Schutzgehäuse weisen den Nachteil auf, dass die Haken als Zurückhaltemittel nicht sicher verhindern können, dass die Kanüle selbsttätig doch wieder austritt. Es ist nämlich nicht ausgeschlossen, dass die Kanüle seitlich entlang der freien Hakenenden wieder aus dem Schutzgehäuse herausgleitet. Dazu trägt auch bei, dass der Einführungsschlitz stets offen bleibt, wodurch weiterhin das Problem vorliegt, dass mitunter kontaminierte Flüssigkeit austreten kann, weil die Kanüle nach der Arretierung mittels der Haken in dem Schutzgehäuse nur von drei Seiten umschlossen wird. Abgesehen davon ist dieses Schutzgehäuse aufgrund der zusätzlichen Zurückhaltemittel bzw. Haken in seiner spritzgießtechnischen Herstellung entsprechend aufwendig.

Der Erfindung liegt daher die Aufgabe zugrunde, eine gattungsgemäße Blutentnahmevorrichtung ohne diese Nachteile zu schaffen und insbesondere mit einer hohen Sicherheitsfunktion für den Benutzer bereitzustellen.

Dese Aufgabe wird erfindungsgemäß dadurch gelöst, dass die freien Enden der Seitenwände des Schutzgehäuses komplementär zu einem sich über die gesamte Gehäuselänge erstreckenden Kanülen-Einführtrichter ausgeformt sind. Es lassen sich hiermit mehrere Vorteile gleichzeitig erreichen. Denn der Einführtrichter fädelt beim Verschwenken bzw. Aufdrücken des Schutzgehäuses die Kanüle zielgenau in das Innere des Schutzgehäuses ein, wo die Kanüle dann allseitig von den Wänden des Schutzgehäuses im Zusammenspiel mit den Wänden des Einführtrichters umschlossen wird. Das Verjüngungs- und damit Durchtrittsende des Einführtrichters kann nämlich sehr eng, deutlich kleiner aus der Kanülendurchmesser bemessen werden, weil sich die Wände des Einführtrichters beim Aufdrücken des Schutzgehäuses auf die Kanüle weiten können, um sich danach dann wieder in ihr eng begrenzte, gegebenenfalls mit Berührung anliegende Ausgangslage zurückzustellen. In dieser verhindert der Einführtrichter dann sicher das ungewollte Austreten der Kanüle, ohne dass dazu im Inneren des Schutzgehäuses unterstützende Zurückhaltemittel vorgesehen werden müssen. Schließlich wird auch ausgeschlossen, dass der Kanüle eventuell anhaftendes, sich ablösendes Blut über den Einführtrichter von innen nach außen gelangen kann.

Ein bevorzugter Vorschlag der Erfindung sieht vor, dass die eine Trichtermündung bildenden freien Wandenden bzw. Trichterwände mit einem dem in das Schutzgehäuse eingetauchten freien Kanülenende zugewandten, als Kanülenabweiser ausgebildeten Mündungskopf versehen sind. Hierzu eignen sich insbesondere mit aufeinander zulaufenden Anschrägungen bzw. -spitzungen, alternativ mit jeweils nur einem Außenradius, stimkantige Vollradien könnten zu einem an dieser Stelle nachteiligen Innentrichter führen, versehene Trichterwände. Sobald das eingeschlossene freie Kanülenende aufgrund äußerer Krafteinwirkung auf einen solchermaßen ausgebildeten Mündungskopf auftrifft, wird es abgewiesen bzw. -gelenkt und kann nicht unerwünscht über den Einführtrichter aus dem Schutzgehäuse heraustreten.

Weitere Merkmale und Einzelheiten der Erfindung ergeben sich aus dem Patentanspruch und der nachfolgenden Beschreibung eines in den Zeichnungen dargestellten Ausführungsbeispiels der Erfindung. Es zeigen:
- Fig. 1: als Einzelheit einer Blutentnahmevorrichtung in einer Gesamtansicht einen Kanülenhalter mit daran angeordnetem Schutzgehäuse, vor dem Aufdrücken des Schutzgehäuses dargestellt;
- Fig. 2: den Kanülenhalter nach Fig. 1 mit verschwenktem, das freie Ende der Kanüle umschließenden Schutzgehäuse, in einem Teillängsschnitt dargestellt; und
- Fig. 3: in einem vergrößerten Maßstab einen Schnitt entlang der Linie III-III von Fig. 2, das vollständige Schutzgehäuse zugrundegelegt.

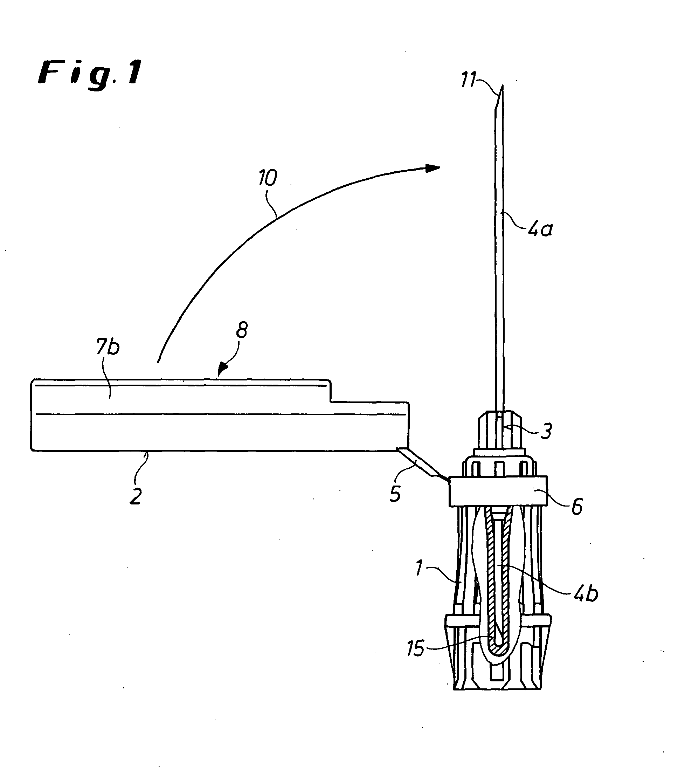

Ein Kanülenhalter 1 nach Fig. 1 wird üblich im Zustand mit - wie dargestellt - ausgeschwenktem bzw. abgeklapptem Schutzgehäuse 2 geliefert, wobei ein freies Kanülenende 4 a seiner Kanüle bzw. im Ausführungsbeispiel Doppelkanüle 3, deren unteres, ebenfalls eine angeschärfte Spitze besitzendes Kanülende 4 b von einem Ventilgummi 15 umschlossen wird, durch eine separate, nicht gezeigte Umhüllung geschützt ist, die vor dem Gebrauch entfernt wird; nach dem Gebrauch wird dann statt dessen das freie Kanülenende 4 a der Doppelkanüle 3 mit dem Schutzgehäuse 2 umschlossen und von diesem eingekammert. Eine als solche bekannte, in ihrer Gesamtheit nicht dargestellte Blutentnahmevorrichtung umfasst den Kanülenhalter 1, der auf ein Blutentnahmeröhrchen bzw. Probenröhrchen aufgesteckt oder mit diesem verschraubt werden kann.

Das Schutzgehäuse 2 ist über ein Band- oder Laschenschamier 5 an einem Haltering 6 befestigt, der am oberen, dem freien Kanülenende 4 a zugewandten Ende des Kanülenhalters 1 angeordnet ist.

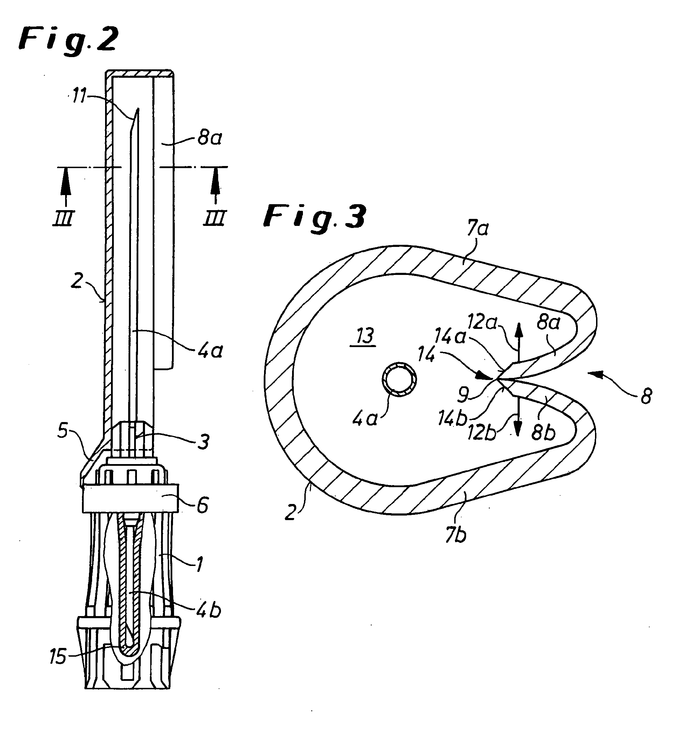

An dem hier ovalen, im Querschnitt im wesentlichen tropfenförmigen Schutzgehäuse 2 ist zwischen den beiden einander gegenüberliegenden Seitenwänden 7a, 7b direkt einstückig an deren oberen, freien Enden als Kanüleneintrittsöffnung ein Einführtrichter 8 ausgebildet (vgl. Fig. 3), der sich ununterbrochen nahezu über die gesamte Länge des Schutzgehäuses 2 eine erstreckt (vgl. die Fig. 1 und 2). Wie sich näher der Fig. 3 entnehmen lässt, besitzen die die freien Enden der Seitenwände 7a, 7b bildenden Trichterwände 8a, 8b gegenüber den Seitenwänden 7a, 7b des Schutzgehäuses 2 eine geringere bzw. sich zur Trichtermündung 9 allmählich verringernde Wanddicke, so dass sie einen elastischen, flexiblen Abschluß der Seitenwände 7a, 7b bilden.

Nach dem Gebrauch der Blutentnahmevorrichtung wird das Schutzgehäuse 2 in Pfeilrichtung 10 gemäß Fig. 1 auf das oben eine angeschärfte Spitze 11 besitzende freie Kanülenende 4 a der Doppelkanüle 3 verschwenkt bzw. aufgedrückt. Damit fädelt sich das freie Kanülenende 4 a über den Einführtrichter 8, dessen Trichterwände 8a, 8b sich dabei zu einer sich temporär weitenden Trichtermündung 9 gegenläufig (vgl. die Pfeile 12a und 12b in Fig. 3) voneinander entfernen, in das Schutzgehäuse 2 ein. Sobald das freie Kanülenende 4 a die Trichtermündung 9 passiert hat, stellen sich die Trichterwände 8a, 8b in zu den Pfeilen 12a, 12b entgegengesetzter Richtung in ihre Ausgangslage zurück, in der die Trichtermündung 9 des Einführtrichters 8 einen Auslaß nach außen sperrt.

Das völlig hochgeschwenkte Schutzgehäuse 2 nimmt danach in seinem allseitig eingeschlossenen Inneren bzw. Kanal 13 das freie Kanülenende 4 a der Doppelkanüle 3 sicher auf. Ein ungewolltes, aufgrund äußerer Krafteinwirkungen selbsttätiges Austreten des freien Kanülenendes 4 a aus den Kanal 13 des Schutzgehäuses 2 wird durch die geschlossene Trichtermündung 9 verhindert.

Das wird noch dadurch besonders unterstützt, dass die Trichterwände 8a, 8b an ihren die Trichtermündung 9 bildenden freien Enden zu einem das freie Kanülen - ende 4 a abweisenden Mündungskopf 14 ausgebildet sind, wozu sie im Ausführungsbeispiel jeweils außenseitig komplementär angespitzt sind. Das begünstigt, dass das freie Kanülenende 4 a, sollte es sich doch einmal der Naht- bzw. Berührungsstelle der Trichterwände 8a, 8b des Einführtrichters 8 annähern und auf diese auftreffen, von den Spitzen 14a, 14b bzw. Wandschrägen, die als Abweiser wirken, des Mündungskopfes 14 nach außen abgelenkt wird. Das freie Kanülenende 4 a ist somit auf jeden Fall ohne zusätzliches Hilfsmittel in Form von Rückhaltehaken oder dergleichen in dem Kanal 13 des Schutzgehäuses 2 eingekammert.

## Patentansprüche

1. Blutentnahmevorrichtung mit einem Kanülenhalter (1), insbesondere für eine Doppelkanüle (3), der ein schwenkbares, bis auf eine zwischen einander gegenüberliegenden Seitenwänden (7a, 7b) vorgesehene Kanüleneintrittsöffnung umfangsgeschlossenes Schutzgehäuse (2) für das freie Kanülenende (4 a) aufweist.
**dadurch gekennzeichnet,**
**daß** die freien Enden (8a, 8b) der Seitenwände (7a, 7b) des Schutzgehäuses (2) komplementär zu einem sich weitestgehend über die gesamte Gehäuselänge erstreckenden Kanülen-Einführtrichter (8) ausgeformt sind.

2. Blutentnahmevorrichtung nach Anspruch 1,
**dadurch gekennzeichnet,**
**daß** die eine Trichtermündung (9) bildenden freien Wandenden bzw. Trichterwände (8a,8b) mit einem dem in das Schutzgehäuse (2) eingetauchten freien Kanülenende (4 a) zugewandten, als Kanülenabweiser (14a, 14b) ausgebildeten Mündungskopf (14) versehen sind.
